(19) Europäisches Patentamt
European Patent Office
Office européen des brevets

(11) Veröffentlichungsnummer : **0 137 418**
**B1**

(12) # EUROPÄISCHE PATENTSCHRIFT

(45) Veröffentlichungstag der Patentschrift : **30.12.86**

(51) Int. Cl.[4] : **C 07 C 131/00**, C 07 C 83/02

(21) Anmeldenummer : **84111511.6**

(22) Anmeldetag : **27.09.84**

(54) **Verfahren zur Herstellung von partiell hydrierten Derivaten von 2-Nitro-1,1,1-trifluoralkanen.**

(30) Priorität : **07.10.83 DE 3336498**

(43) Veröffentlichungstag der Anmeldung : **17.04.85 Patentblatt 85/16**

(45) Bekanntmachung des Hinweises auf die Patenterteilung : **30.12.86 Patentblatt 86/52**

(84) Benannte Vertragsstaaten : **CH DE FR GB IT LI**

(56) Entgegenhaltungen :
**CH-A- 325 080**
**CH-A- 344 073**
**DE-A- 1 186 050**
**DE-A- 1 193 032**
**DE-C- 917 426**

(73) Patentinhaber : **BAYER AG**
**Konzernverwaltung RP Patentabteilung**
**D-5090 Leverkusen 1 Bayerwerk (DE)**

(72) Erfinder : **Baasner, Bernd, Dr.**
**Mozartstrasse 41**
**D-5090 Leverkusen 1 (DE)**
Erfinder : **Ziemann, Heinz, Dr.**
**Am Wiesenberg 10**
**D-5653 Leichlingen 1 (DE)**
Erfinder : **Klauke, Erich, Dr.**
**Eichendorffweg 8**
**D-5068 Odenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 137 418 B1

**Beschreibung**

Die vorliegende Erfindung betrifft ein neues Verfahren zur Herstellung partiell hydrierter Derivate von 2-Nitro-1,1,1-trifluoralkanen, insbesondere ein Verfahren zur Herstellung von 2-Oximino- und 2-Hydroxylamino-1,1,1-trifluoralkanen.

Es ist bereits bekannt, Fluor enthaltende Oxime durch Umsetzung der entsprechenden Aldehyde oder Ketone mit Hydroxylamin herzustellen (siehe z. B. J. Org. Chem. *31*, 964 (1966)). Die hierbei als Ausgangsstoffe benötigten Aldehyde und Ketone sind aber nur nach aufwendigen Verfahren und in mäßigen Ausbeuten zugänglich (siehe Bull. Sci. Acad. Roy. Belg. *13*, 175 (1927) und J. Am. Chem. Soc. *72*, 3371 (1950)), sodaß dieser Weg für eine technische Anwendung nicht in Frage kommt.

Aus den Oximen können durch Reduktion mit Borwasserstoff Hydroxylamine erhalten werden (siehe J. Med. Chem. *13*, 238 (1970)). Dieses Verfahren ist für eine technische Anwendung ebenfalls nicht geeignet, da Borwasserstoffe selbstentzündlich sind und nur mit sehr aufwendigen Sicherheitsmaßnahmen gehandhabt werden können.

Bei der Hydrierung von fluorfreien Nitroalkanen können die entsprechenden Oxime und Hydroxylamine nur in mäßigen Ausbeuten, bei nur aufwendig einzuhaltenden Reaktionsbedingungen und in nur unzureichenden Selektivitäten erhalten werden. Es fallen Gemische an, die neben den gewünschten Oximen und/oder Hydroxylaminen noch unumgesetztes Nitroalkan und vollständig hydrierte Produkte (Amine) enthalten (siehe Houben-Weyl, Methoden der organischen Chemie, Band IV, 1c, Teil I, Seite 496-497, Georg Thieme Verlag, Stuttgart, 1980). Bei der katalytischen Hydrierung von Oximen fallen als Hauptprodukt die entsprechenden Amine und nur in geringen Mengen die Hydroxylamine an (siehe Houben-Weyl, a.a.O.S. 250-252). In jüngster Zeit wird sogar ausgeschlossen, daß bei der Hydrierung von Nitroethan ein Oxim als Zwischenprodukt auftritt (siehe J. of Catalysis *82*, 56-65 (1983)). Hinzu kommt noch, daß bei der Hydrierung von fluorhaltigen Nitroalkanen mit der Abspaltung von Fluorwasserstoff zu rechnen ist.

Es gibt also bisher kein technisch brauchbares Verfahren zur Herstellung von partiell hydrierten Derivaten von 2-Nitro-1,1,1-trifluoralkanen, insbesondere von 2-Oximino- und 2-Hydroxylamino-1,1,1-trifluoralkanen, und aus den Gegebenheiten bei der Hydrierung von fluorfreien Nitroalkanen kann nicht erwartet werden, daß eine Herstellung von partiell hydrierten Derivaten von 2-Nitro-1,1,1-trifluoralkanen auf der Basis einer partiellen katalytischen Hydrierung möglich sein könnte.

Es wurde nun ein Verfahren zur Herstellung von partiell hydrierten Derivaten von 2-Nitro-1,1,1-trifluoralkanen gefunden, das dadurch gekennzeichnet ist, daß man 2-Nitro-1,1,1-trifluoralkane katalytisch hydriert bis maximal 2,2 Mole Wasserstoff pro Mol 2-Nitro-1,1,1-trifluoralkan umgesetzt worden sind.

Das erfindungsgemäße Verfahren eignet sich insbesondere zur Herstellung von 2-Oximino- und 2-Hydroxylamino-1,1,1-trifluoralkanen. Zur Herstellung von 2-Oximino-1,1,1-trifluoralkanen führt man die katalytische Hydrierung vorzugsweise durch bis 0,7 bis 1,2, besonders bevorzugt 0,8 bis 1,1, ganz besonders bevorzugt 1 Mol Wasserstoff pro Mol 2-Nitro-1,1,1-trifluoralkan umgesetzt worden ist. Zur Herstellung von 2-Hydroxylamino-1,1,1-trifluoralkanen führt man die katalytische Hydrierung vorzugsweise durch bis 1,9 bis 2,2, besonders bevorzugt 1,95 bis 2,2, ganz besonders bevorzugt 2 Mole Wasserstoff pro Mol 2-Nitro-1,1,1-trifluoralkan umgesetzt worden sind.

In das erfindungsgemäße Verfahren können beispielsweise 2-Nitro-1,1,1-trifluoralkane der Formel (I)

$$CF_3 - \underset{NO_2}{CH} - R \qquad (I)$$

in der R für Wasserstoff, einen Alkylrest mit 1 bis 15 C-Atomen oder einen Cycloalkylrest mit 5 bis 8 C-Atomen steht, eingesetzt werden. Vorzugsweise steht in Formel (I) R für Wasserstoff oder einen Alkylrest mit 1 bis 8 C-Atomen.

Die in das erfindungsgemäße Verfahren einzusetzenden 2-Nitro-1,1,1-trifluoralkane können beispielsweise durch konjugierte Nitrofluorierung aus den entsprechenden Olefinen hergestellt werden (siehe Dokl. Akad. Nauk. SSR *149*, 2222-2225 (1963) (engl.) und Izvest. Akad. Nauk. SSR 1963, 1794-1797 (engl.), sowie die demgegenüber verbesserten Verfahren gemäß den DE-OS'en 3 305 201 und 3 305 202).

Das erfindungsgemäße Verfahren kann in Gegenwart oder in Abwesenheit von Lösungsmitteln durchgeführt werden. Im allgemeinen ist es vorteilhaft, in Gegenwart eines Lösungsmittels zu arbeiten, da dann eine bessere Kontrolle der exotherm verlaufenden Hydrierung möglich ist.

Als Lösungsmittel kommen beispielsweise inerte organische Lösungsmittel in Frage. Geeignet sind beispielsweise Alkohole wie Methanol, Ethanol, Ethylenglykol und Diethylenglykol, Ether wie Dioxan, Tetrahydrofuran, Ethylenglykolmonomethylether, Ethylenglykolmonoethylether, Ethylenglykoldimethylether, Diethylenglykolmonomethylether und Diethylenglykoldimethylether, gesättigte Kohlenwasserstoffe wie Cyclohexan oder Ester wie Essigsäureethylester.

Ein sehr gut zu kontrollierender Reaktionsverlauf läßt sich durch den Zusatz von Wasser zu den organischen Lösungsmitteln erreichen. Die Hydrierung kann auch in Wasser selbst durchgeführt werden,

jedoch ist hierbei die erzielbare Selektivität im allgemeinen niedriger als im organischen Lösungsmittel. Bevorzugt wird als Lösungsmittel Methanol eingesetzt, dem bis zu 20 Gew.-% Wasser zugesetzt sein können. In diesem Fall ist im allgemeinen eine problemlose destillative Abtrennung der Lösungsmittel von den Hydrierprodukten möglich.

Das erfindungsgemäße Verfahren kann in Reaktionsapparaturen durchgeführt werden, die für Hydrierungen bei Normaldruck und/oder Hydrierungen unter erhöhtem Druck geeignet sind. Geeignete Materialien für die Reaktionsapparaturen sind beispielsweise Glas, Emaille, Stahl oder Edelstahl.

Der Wasserstoffdruck, unter dem die Hydrierung durchgeführt wird, kann in weiten Grenzen variieren. Die Reaktion verläuft im allgemeinen umso selektiver, je geringer der Druck ist. Bevorzugt ist ein Druck von 1 bis 20 bar, besonders bevorzugt von 1,2 bis 8 bar.

Die Reaktionstemperatur kann ebenfalls in weiten Grenzen variiert werden und beispielsweise zwischen 0 und 120 °C liegen. Besonders bevorzugt sind Temperaturen zwischen 10 und 80 °C, ganz besonders bevorzugt solche zwischen 20 und 60 °C.

Die Hydrierung verläuft stufenweise. Wenn man die Hydrierung jeweils dann abbricht, wenn die berechnete Menge an Wasserstoff umgesetzt worden ist, das ist 1 Mol Wasserstoff pro Mol 2-Nitro-1,1,1-trifluoralkan bei der Herstellung des Oxims und 2 Mol Wasserstoff pro Mol 2-Nitro-1,1,1-trifluoralkan bei der Synthese des Hydroxylamins, wird das jeweilige Produkt in optimaler Selektivität und Ausbeute erhalten. Wenn man auf diese Weise 2-Oximino-1,1,1-trifluoralkane herstellt, sind jeweils nur geringe Mengen an unverändertem Ausgangsmaterial und weiter hydrierten Produkten (Hydroxylamine und Amine) im Reaktionsprodukt nachweisbar. Wenn man auf diese Weise 2-Hydroxylamino-1,1,1-trifluoralkane herstellt sind im Reaktionsprodukt jeweils nur Spuren des entsprechenden Oxims nachzuweisen. Die Bildung von Aminen wird im allgemeinen nur beobachtet, wenn die Wasserstoffdrucke über 5 bar liegen. Im Druckbereich von 5 bis 20 bar ist die Amin-Bildung aber noch relativ gering. Erst bei Drucken von über 20 bar entstehen größere Mengen von Aminen.

Die für das erfindungsgemäße Verfahren erforderliche Reaktionszeit ist abhängig von der Reaktionsgeschwindigkeit, dem Wasserstoffpartialdruck, der Intensität der Durchmischung des Reaktionsgemisches und von der Aktivität und Konzentration des Hydrierkatalysators. Im allgemeinen liegt die erforderliche Reaktionszeit im Bereich von 15 Minuten bis zu mehreren Stunden.

Das erfindungsgemäße Verfahren wird in Gegenwart von Hydrierkatalysatoren durchgeführt. Geeignete Hydrierkatalysatoren sind beispielsweise solche, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielsweise um Oxide, Hydroxide und/oder Oxidhydrate handeln. Zusätzlich können die Metalle Kupfer, Vanadin, Molybdän, Chrom und/oder Mangan, sowie Verbindungen dieser Metalle zugegen sein.

Die Hydrierkatalysatoren können ausschließlich oder überwiegend aus Wasserstoff-übertragenden Substanzen bestehen, diese können aber auch auf Trägermaterialien aufgebracht sein. Als Trägermaterialien für die Wasserstoff-übertragenden Substanzen kommen beispielsweise in Frage : anorganische Materialien wie Kieselgur, Kieselsäure, Aluminiumoxide, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest, Aktivkohle oder Bariumsulfat, aber auch organische Materialien, beispielsweise natürlich vorkommende oder synthetische Verbindungen mit hohen Molekulargewichten wie Seide, Polyamide, Polystyrole, Zellstoff oder Polyurethane. Bevorzugt sind anorganische Trägermaterialien. Das Trägermaterial kann beispielsweise in Form von Kugeln, Strängen, Fäden, zylindern, Polygonen oder in Pulverform vorliegen.

Derartige Trägerkatalysatoren können im allgemeinen 0,5 bis 50 Gew.-%, vorzugsweise 1 bis 10 Gew.-% der wasserstoffübertragenden Substanz, bezogen auf die Gesamtmasse des Trägerkatalysators, enthalten. Die wasserstoffübertragende Substanz kann dabei homogen im Trägermaterial verteilt sein, bevorzugt sind jedoch Katalysatoren, in deren äußerer Schicht oder auf deren Oberfläche die wasserstoffübertragende Substanz abgelagert ist. Die Herstellung und die Formgebung von Katalysatoren, die im erfindungsgemäßen Verfahren Verwendung finden können, kann in bekannter Weise erfolgen (siehe beispielsweise Houben-Weyl, Methoden der organischen Chemie, Band IV, 1c, Teil I, S. 16-26, Georg Thieme Verlag, Stuttgart 1980).

Bevorzugte Trägerkatalysatoren sind Ruthenium auf Kohle, Ruthenium auf Aluminiumoxid, Rhodium auf Kohle, Rhodium auf Aluminiumoxid, Palladium auf Kohle, Palladium auf Aluminiumoxid, Palladium auf Calciumcarbonat, Palladium auf Bariumsulfat, Palladium auf Kieselsäure, Platin auf Kohle und Platin auf Aluminiumoxid.

Bevorzugte Hydrierkatalysatoren, die ausschließlich oder überwiegend aus wasserstoffübertragender Substanz bestehen, sind beispielsweise oxidische Katalysatoren wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit Alkalihydriden, Alkaliboranaten, Metallalkylen, Hydrazin, Formaldehyd, Wasserstoff oder elektropositiveren Metallen herstellbare Schwarzkatalysatoren, wie Palladium/Schwarz, Platin/Schwarz und Rhodium/Schwarz.

Besonders bevorzugte Katalysatoren für das erfindungsgemäße Verfahren sind Palladium auf Kohle, Palladium auf Aluminiumoxid, Palladium auf Kieselsäure und Palladium auf Calciumcarbonat.

Insbesondere für die Herstellung von 2-Hydroxylamino-1,1,1-trifluoralkanen können auch Skelettkatalysatoren vom Raney-Typ eingesetzt werden, wobei Raney-Nickel bevorzugt ist.

Der Hydrierkatalysator kann in das erfindungsgemäße Verfahren beispielsweise in Mengen von 0,05 bis 2,5 Gew.-% wasserstoffübertragende Substanz, bezogen auf das Gesamtgewicht des Reaktionsgemisches, eingesetzt werden. Vorzugsweise beträgt diese Menge 0,1 bis 1 Gew.-%.

Zur Durchführung des erfindungsgemäßen Verfahrens können auch Gemische aus zwei oder mehreren der genannten Hydrierkatalysatoren verwendet werden.

Die katalytische Aktivität der Hydrierkatalysatoren bleibt bei der Durchführung des erfindungsgemäßen Verfahrens im allgemeinen weitgehend erhalten, so daß diese bei diskontinuierlicher Arbeitsweise wiederholt eingesetzt werden und bei kontinuierlicher Arbeitsweise längere Zeit in Gebrauch bleiben können.

In einer einfachen diskontinuierlichen Ausführungsform kann das erfindungsgemäße Verfahren beispielsweise wie folgt durchgeführt werden : Ein mit einer Rühr- oder Mischeinrichtung versehener, temperierbarer Autoklav wird mit dem einzusetzenden 2-Nitro-1,1,1-trifluoralkan, dem Hydrierkatalysator und einem Lösungsmittel beschickt. Danach wird Wasserstoff bis zum gewünschten Druck aufgedrückt und das Gemisch unter intensiver Durchmischung auf die gewünschte Reaktionstemperatur erhitzt. Der Reaktionsverlauf läßt sich leicht durch Messung des Wasserstoffverbrauchs verfolgen. Während der Reaktion verbrauchter Wasserstoff kann kontinuierlich oder diskontinuierlich nachdosiert werden. Die Hydrierung wird abgebrochen, wenn die gewünschte Menge Wasserstoff aufgenommen worden ist. Der Abbruch der Hydrierung kann durch Abkühlung, Beendigung der Durchmischung, Entspannung und/oder Beseitigung der Wasserstoffatmosphäre erfolgen. Die Aufarbeitung des Reaktionsgemisches kann beispielsweise so erfolgen, daß man zunächst den Katalysator abfiltriert, danach das Lösungsmittel abdestilliert und die verbleibenden Reaktionsprodukte durch Destillation oder Kristallisation reinigt. Das erfindungsgemäße Verfahren kann auch kontinuierlich durchgeführt werden.

Die erfindungsgemäß herstellbaren partiell hydrierten Derivate von 2-Nitro-1,1,1-trifluoralkanen, insbesondere 2-Oximino- und 2-Hydroxylamino-1,1,1-trifluoralkane, sind wertvolle Ausgangsstoffe zur Herstellung von biologisch aktiven Verbindungen, beispielsweise von insektizid wirksamen Oximocarbamaten.

Mit dem erfindungsgemäßen Verfahren gelingt es partiell hydrierte Derivate von 2-Nitro-1,1,1-trifluoralkanen, insbesondere 2-Oximino-1,1,1-trifluoralkane und 2-Hydroxylamino-1,1,1-trifluoralkane, in hohen Selektivitäten, guten Ausbeuten und hohen Reinheiten herzustellen. Im Hinblick auf die eingangs geschilderten Gegebenheiten bei der Hydrierung von fluorfreien Nitroalkanen ist es als ausgesprochen überraschend anzusehen, daß dies möglich ist.

Die folgenden Beispiele erläutern die vorliegende Erfindung.

## Beispiele

### Beispiel 1

Hydrierung von 2-Nitro-1,1,1-trifluorpropan zu 2-Oximino-1,1,1-trifluorpropan.

In einem Hydrierautoklaven wurden 71,5 g (0,5 Mol) 2-Nitro-1,1,1-trifluorpropan in 40 ml Methanol in Gegenwart von 5 g Palladium auf Calciumcarbonat (5 %ig) bei 2 bar Wasserstoffdruck und 25 bis 30 °C bis zur Aufnahme von 0,5 Mol Wasserstoff hydriert. Anschließend wurde der Katalysator abfiltriert und mit Methanol nachgewaschen. Eine gaschromatographische Untersuchung des Filtrates ergab, daß darin 2-Nitro-1,1,1-trifluorpropan, 2-Hydroxylamino-1,1,1-trifluorpropan und 2-Oximino-1,1,1-trifluorpropan im Gewichtsverhältnis von 5 : 7 : 88 vorhanden war.

Das Filtrat wurde fraktioniert destilliert und dabei mit einem Siedepunkt von 104 bis 106 °C 49,4 g (78 % der Theorie) an 2-Oximino-1,1,1-trifluorpropan erhalten.

### Beispiel 2

Hydrierung von 2-Nitro-1,1,1-trifluorpropan zu 2-Hydroxylamino-1,1,1-trifluorpropan.

In einem Hydrierautoklaven wurden 71,5 g (0,5 Mol) 2-Nitro-1,1,1-trifluorpropan in 40 ml 95 %igem wäßrigem Methanol in Gegenwart von 0,5 g Palladium-Schwarz bei 2 bar Wasserstoffdruck und 45 bis 50 °C bis zur Aufnahme von einem Mol Wasserstoff hydriert. Anschließend wurde der Katalysator abfiltriert, mit Methanol nachgewaschen und das Filtrat im Wasserstrahlvakuum bis zur Trockene eingeengt. Es hinterblieben 60,6 g (94 % der Theorie) 2-Hydroxylamino-1,1,1-trifluorpropan, dessen gaschromatographisch ermittelte Reinheit 98,7 % betrug.

Durch Umkristallisation einer kleinen Probe des erhaltenen 2-Hydroxylamino-1,1,1-trifluorpropans aus n-Hexan wurden farblose Nadeln mit einem konstanten Schmelzpunkt von 88,5 bis 89 °C erhalten.

### Beispiel 3

Hydrierung von 2-Nitro-1,1,1-trifluorethan zu 2-Oximino-1,1,1-trifluorethan.

In einem Hydrierautoklaven wurden 64,5 g (0,5 Mol) 2-Nitro-1,1,1-trifluorethan in 50 ml Methanol in Gegenwart von 5 g Palladium auf Calciumcarbonat (5 %ig) bei 1,9 bar Wasserstoffdruck und 25 bis 30 °C bis zur Aufnahme von 0,5 Mol Wasserstoff hydriert. Anschließend wurde der Katalysator abfiltriert und mit Methanol nachgewaschen. Die gaschromatographische Untersuchung des Filtrats ergab, daß darin 2-Nitro-1,1,1-trifluorethan, 2-Hydroxylamino-1,1,1-trifluorethan und 2-Oximino-1,1,1-trifluorethan im Gewichtsverhältnis von 10 : 14 : 76 vorhanden waren.

Durch fraktionierte Destillation des Filtrates wurden mit einem Siedepunkt von 89 bis 94 °C 34,5 g (61 % der Theorie) 2-Oximino-1,1,1-trifluorethan erhalten, dessen Reinheit nach gaschromatographischer Untersuchung 96,3 %ig war.

Beispiel 4

Hydrierung von 2-Nitro-1,1,1-trifluorethan zu 2-Hydroxylamino-1,1,1-trifluorethan.

In einem Hydrierautoklaven wurden 64,5 g (0,5 Mol) 2-Nitro-1,1,1-trifluorethan in 40 ml 95 %igen wäßrigen Methanol in Gegenwart von 8 g Raney-Nickel bei einem Wasserstoffdruck von 2 bar bis zur Aufnahme von einem Mol Wasserstoff hydriert. Anschließend wurde vom Katalysator abfiltriert und mit Methanol nachgewaschen. Das Filtrat wurde über einem Molekularsieb getrocknet und das Methanol über eine 10 cm-Füllkörperkolonne abdestilliert. Es hinterblieben 53,5 g (93 % der Theorie) 2-Hydroxylamino-1,1,1-trifluorethan mit einem Schmelzpunkt von 78 bis 79 °C, dessen Reinheit nach gaschromatographischer Untersuchung 96,5 % betrug.

**Patentansprüche**

1. Verfahren zur Herstellung von partiell hydrierten Derivaten von 2-Nitro-1,1,1-trifluoralkanen, dadurch gekennzeichnet, daß man 2-Nitro-1,1,1-trifluoralkane katalytisch hydriert bis maximal 2,2 Mole Wasserstoff pro Mol 2-Nitro-1,1,1-trifluoralkan umgesetzt worden sind.

2. Verfahren nach Anspruch 1, dadurch gekennzeichnet, daß man 2-Nitro-1,1,1-trifluoralkane der Formel

$$CF_3 - \underset{NO_2}{\underset{|}{CH}} - R$$

einsetzt, in der R für Wasserstoff, einen Alkylrest mit 1 bis 15 C-Atomen oder für einen Cycloalkylrest mit 5 bis 8 C-Atomen steht.

3. Verfahren nach Ansprüchen 1 bis 2, dadurch gekennzeichnet, daß man in Gegenwart eines Lösungsmittels arbeitet.

4. Verfahren nach Ansprüchen 1 bis 3, dadurch gekennzeichnet, daß man bei Drucken im Bereich 1 bis 20 bar arbeitet.

5. Verfahren nach Ansprüchen 1 bis 4, dadurch gekennzeichnet, daß man bei Drücken im Bereich 1, 2 bis 8 bar arbeitet.

6. Verfahren nach Ansprüchen 1 bis 5, dadurch gekennzeichnet, daß man bei Temperaturen im Bereich 0 bis 120 °C arbeitet.

7. Verfahren nach Ansprüchen 1 bis 6, dadurch gekennzeichnet, daß man bei Temperaturen im Bereich 10 bis 80 °C arbeitet.

8. Verfahren nach Ansprüchen 1 bis 7, dadurch gekennzeichnet, daß man in Gegenwart von Hydrierkatalysatoren arbeitet, die aus Metallen und/oder -Verbindungen von Elementen der VIII. Nebengruppe des Periodensystems der Elemente nach Mendelejew bestehen oder diese enthalten.

9. Verfahren nach Ansprüchen 1 bis 8, dadurch gekennzeichnet, daß man zur Herstellung von 2-Oximino-1,1,1-trifluoralkanen die Hydrierung durchführt, bis 0,7 bis 1,2 Mole Wasserstoff pro Mol 2-Nitro-1,1,1-trifluoralkan umgesetzt worden sind.

10. Verfahren nach Ansprüchen 1 bis 8, dadurch gekennzeichnet, daß man zur Herstellung von 2-Hydroxylamino-1,1,1-trifluoralkanen die Hydrierung durchführt, bis 1,9-2,2 Mole Wasserstoff pro Mol 2-Nitro-1,1,1-trifluoralkan umgesetzt worden sind.

**Claims**

1. Process for the preparation of partially hydrogenated derivates of 2-nitro-1,1,1-trifluoroalkanes, characterised in that 2-nitro-1,1,1-trifluoroalkanes are catalytically hydrogenated until a maximum of 2.2 moles of hydrogen have reacted per mole of 2-nitro-1,1,1-trifluoroalkane.

2. Process according to Claim 1, characterised in that 2-nitro-1,1,1-trifluoroalkanes of the formula

$$CF_3 - \underset{NO_2}{\underset{|}{CH}} - R$$

are used, in which R represents hydrogen, an alkyl radical with 1 to 15 C atoms or a cycloalkyl radical with 5 to 8 C atoms.

3. Process according to Claims 1 to 2, characterised in that it is carried out in the presence of a solvent.

4. Process according to Claims 1 to 3, characterised in that it is carried out under pressures in the range 1 to 20 bar.

5. Process according to Claims 1 to 4, characterised in that it is carried out under pressures in the range 1.2 to 8 bar.

6. Process according to Claims 1 to 5, characterised in that it is carried out at temperatures in the range 0 to 120 °C.

7. Process according to Claims 1 to 6, characterised in that it is carried out at temperatures in the range 10 to 80 °C.

8. Process according to Claims 1 to 7, characterised in that it is carried out in the presence of hydrogenation catalysts which consist of or contain metals and/or compounds of elements of subgroup VIII of the periodic system of elements according to Mendeleeff.

9. Process according to Claims 1 to 8, characterised in that, to prepare 2-oximino-1,1,1-trifluoroalkanes, the hydrogenation is carried out until 0.7 to 1.2 moles of hydrogen have reacted per mole of 2-nitro-1,1,1-trifluoroalkane.

10. Process according to Claims 1 to 8, characterised in that, to prepare 2-hydroxylamino-1,1,1-trifluoroalkanes, the hydrogenation is carried out until 1.9-2.2 moles of hydrogen have reacted per mole of 2-nitro-1,1,1-trifluoroalkane.

## Revendications

1. Procédé de production de dérivés partiellement hydrogénés de 2-nitro-1,1,1-trifluoralcanes, caractérisé en ce qu'on hydrogène catalytiquement des 2-nitro-1,1,1-trifluoralcanes jusqu'à ce qu'un maximum de 2,2 moles d'hydrogène par mole de 2-nitro-1,1,1-trifluoralcane ait réagi.

2. Procédé suivant la revendication 1, caractérisé en ce qu'on utilise des 2-nitro-1,1,1-trifluoralcanes de formule

$$CF_3 - \underset{NO_2}{\underset{|}{CH}} - R$$

dans laquelle R désigne l'hydrogène, un reste alkyle ayant 1 à 15 atomes de carbone ou un reste cycloalkyle ayant 5 à 8 atomes de carbone.

3. Procédé suivant les revendications 1 et 2, caractérisé en ce qu'on opère en présence d'un solvant.

4. Procédé suivant les revendications 1 à 3, caractérisé en ce qu'on opère à des pressions comprises dans l'intervalle de 1 à 20 bars.

5. Procédé suivant les revendications 1 à 4, caractérisé en ce qu'on opère à des pressions comprises dans l'intervalle de 1,2 à 8 bars.

6. Procédé suivant les revendications 1 à 5, caractérisé en ce qu'on opère à des températures comprises dans l'intervalle de 0 à 120 °C.

7. Procédé suivant les revendications 1 à 6, caractérisé en ce qu'on opère à des températures comprises dans l'intervalle de 10 à 80 °C.

8. Procédé suivant les revendications 1 à 7, caractérisé en ce qu'on opère en présence de catalyseurs d'hydrogénation qui sont constitués par des métaux et/ou des composés métalliques d'éléments du sous-groupe VIII du système de Classification Périodique des Eléments selon Mendelejew, ou qui en contiennent.

9. Procédé suivant les revendications 1 à 8, caractérisé en ce que pour la production de 2-oximino-1,1,1-trifluoralcanes, on conduit l'hydrogénation jusqu'à ce que 0,7 à 1,2 mole d'hydrogène par mole de 2-nitro-1,1,1-trifluoralcane ait réagi.

10. Procédé suivant les revendications 1 à 8, caractérisé en ce que pour la production de 2-hydroxylamino-1,1,1-trifluoralcanes, on conduit l'hydrogénation jusqu'à ce que 1,9 à 2,2 moles d'hydrogène par mole de 2-nitro-1,1,1-trifluoralcane aient réagi.